# FASCICULE DE BREVET EUROPEEN

(11) **EP 3 758 578 B1**
(45) Date de publication et mention de la délivrance du brevet: **14.09.2022**
(21) Numéro de dépôt: 19711144.6
(22) Date de dépôt: 26.02.2019
(51) Int. Cl.: A61B 3/08, A61F 9/02, G02B 27/01

(54) **DISPOSITIF D'EXPLORATION DU SYSTEME VISUEL**
VORRICHTUNG ZUR UNTERSUCHUNG DES VISUELLEN SYSTEMS
DEVICE FOR EXPLORING THE VISUAL SYSTEM

(30) Priorité: 28.02.2018 FR 1800175
(43) Date de publication de la demande: 06.01.2021
(73) Titulaire: Université de Lorraine, 54052 Nancy (FR)
(72) Inventeur: SCHWANN, Raymund, 54600 Villiers-les-Nancy (FR); LOUIS-DORR, Valérie, 54690 Lay Saint Christophe (FR); SCHWITZER, Thomas, 54000 Nancy (FR)
(74) Mandataire: Boüan du Chef du Bos, Louis-Paterne
(86) Numéro de dépôt international: PCT/FR2019/000023
(87) Numéro de publication internationale: WO 2019/166704

(56) Documents cités:
- CA-A1- 2 824 972
- KR-B1- 101 318 987
- US-A1- 2017 150 897

## Description

### Domaine de l'invention

La présente invention concerne un dispositif permettant de réaliser des explorations du système visuel.

En particulier, la présente invention concerne un tel dispositif mettant en œuvre un équipement de visualisation portable.

### Art antérieur

Différents types d'exploration du système visuel sont connus. Ces explorations sont souvent utilisées pour réaliser des examens ophtalmologiques, pour détecter différentes caractéristiques physiologiques oculaires d'une personne, ou pour explorer le système nerveux central de la personne. Ces explorations peuvent ainsi être utilisées pour la recherche, ou pour permettre le diagnostic de différentes pathologies.

Parmi ces explorations du système visuel, on connaît notamment l'électrorétinogramme, souvent désigné par l'acronyme ERG, qui est un examen électrophysiologique permettant notamment de diagnostiquer certaines pathologies fonctionnelles de la rétine ou certains troubles du système nerveux central.

La réalisation d'un tel examen est faite dans des services spécialisés de neurophysiologie clinique ou d'ophtalmologie, à l'aide d'un dispositif appelé électrorétinographe.

Pour que les pupilles de la personne présentent l'état de dilatation nécessaire à la réalisation de l'examen, celui-ci est placé dans l'obscurité pendant une période d'adaptation de vingt à trente minutes, avant qu'un opérateur ne pose sur son visage, grâce à un éclairage de faible puissance, des électrodes nécessaires à l'examen. Ces électrodes comprennent une ou plusieurs électrodes de mesure, qui peuvent être posées sur la cornée, sur la peau de la paupière inférieure, dans le cul-de-sac conjonctival ou sous la paupière inférieure, et des électrodes de référence, qui sont habituellement posées sur le front, au canthus et au lobe de chaque oreille.

Après la pose de ces électrodes, la personne est placée devant l'électrorétinographe, qui est un dispositif permettant l'émission de stimulations telles que des flashs lumineux ou des stimulations lumineuses à géométrie variable, de préférence en champ total, et de mesurer, par le biais des différentes électrodes, la réponse électrophysiologique de la rétine à ces stimulations lumineuses.

Un tel examen est coûteux, et n'est généralement réalisé qu'en milieu hospitalier ou en centre spécialisé. En effet, l'électrorétinographe est un dispositif volumineux et onéreux, qui doit être placé dans l'obscurité pendant toute la durée de l'examen. Il est également nécessaire, pour pratiquer cet examen, de prévoir une pièce réservée à l'adaptation de la personne à l'obscurité. Enfin, une installation électrique spécifique doit également être mise en place pour le branchement de cet électrorétinographe.

En conséquence, les électrorétinogrammes ne sont pratiqués que pour des indications spécifiques. Il serait pourtant avantageux de pratiquer plus couramment ces examens, afin de permettre une détection plus précoce ou systématique de certaines affections rétiniennes, voire de certaines affections neurologiques ou psychiques.

Le document US 2017/0017083 décrit l'utilisation d'un équipement de visualisation de réalité augmentée pour réaliser une série d'examens ophtalmologiques, parmi lesquels l'électrorétinogramme. Cependant, ce document ne décrit pas les moyens et processus à mettre en oeuvre pour réaliser effectivement un tel examen dans de bonnes conditions.

Le document CA-A-2 824 972 qui est considéré comme étant l'état de la technique le plus proche de l'objet de la revendication 1, décrit un dispositif d'exploration du système visuel selon le préambule de la revendication 1.

### Objectifs de l'invention

La présente invention a pour objectif de pallier ces inconvénients de l'art antérieur.

En particulier, la présente invention a pour objectif de permettre la réalisation d'explorations du système visuel dans des conditions plus aisées, et pour un coût inférieur aux solutions de l'art antérieur.

L'invention a particulièrement pour objectif de permettre la réalisation d'électrorétinogrammes dans des conditions adaptées et parfaitement maitrisées.

### Exposé de l'invention

Ces objectifs, ainsi que d'autres qui apparaîtront plus clairement par la suite, sont atteints à l'aide d'un dispositif d'exploration du système visuel, comprenant un équipement de visualisation portable comportant au moins un écran de visualisation destiné à être placé face à au moins un œil d'une personne. Selon l'invention, cet équipement de visualisation comprend des lèvres d'obturation entourant au moins ledit ou l'un desdits écrans de visualisation, et présentant un bord libre destiné à être appliqué contre la peau de la personne, autour de son œil ou de ses yeux faisant face audit écran, ledit bord libre comprenant au moins une électrode de mesure du potentiel électrique, destinée à être appliquée contre la peau de la personne, et le dispositif comprend au moins une électrode libre de mesure du potentiel électrique, reliée, par un fil de connexion, à un connecteur porté par ledit équipement de visualisation, entre l'un desdits écrans de visualisation et ledit bord libre des lèvres d'obturation entourant cet écran.

Le dispositif selon l'invention permet ainsi à la fois d'isoler parfaitement les yeux de la personne de la lumière extérieure, de réaliser des stimulations lumineuses devant ces yeux, et de mesurer les réponses électrophysiologiques à ces stimulations. Il permet donc de réaliser, particulièrement simplement, un électrorétinogramme.

Selon un mode de réalisation avantageux, l'équipement de visualisation comprend deux écrans de visualisation, destinés chacun à être placé face à l'un des yeux de ladite personne, et lesdites lèvres d'obturation entourent chacun desdits écrans.

Il est ainsi possible de réaliser des stimulations lumineuses différenciées pour les deux yeux, chaque œil étant isolé des stimulations destinées à l'autre œil.

Avantageusement, les lèvres d'obturation comprennent des moyens de maintien de lentilles optiques devant au moins un desdits écrans.

Il est ainsi possible de réaliser facilement des observations du système visuel de personnes nécessitant des corrections de leur vue.

Avantageusement, le dispositif comprend une sangle de maintien dudit équipement de visualisation, destinée à être passée à l'arrière de la tête de ladite personne, ladite sangle de maintien portant au moins une électrode de mesure du potentiel électrique.

Il est ainsi possible de mesurer facilement la réponse électrophysiologique du cortex occipital aux stimulations lumineuses, pendant la réalisation de l'électrorétinogramme.

Selon un mode de réalisation avantageux, ledit connecteur est porté par ledit équipement de visualisation par l'intermédiaire d'un fil de longueur variable.

L'installation des électrodes libres sur la personne, le branchement de ces électrodes à l'équipement de visualisation et l'installation de l'équipement de visualisation peuvent être ainsi facilités.

Préférentiellement, le dispositif comprend un système d'acquisition apte à synchroniser les données mesurées par les électrodes avec les stimulations visuelles produites par ledit ou lesdits écrans.

Avantageusement, ce système d'acquisition comporte des moyens d'amplification des signaux mesurés par les électrodes, des moyens de filtrage de ces signaux, et des moyens de conversion de ces signaux analogiques en des signaux numériques.

Selon une caractéristique avantageuse, le dispositif comprend une sortie audio apte à communiquer des instructions à ladite personne.

Cette sortie audio est par exemple constituée par des haut-parleurs.

Avantageusement, le dispositif comprend au moins un périphérique d'entrée.

Un tel périphérique peut par exemple être constitué par une manette de commande, tenue par la personne portant l'équipement de visualisation, ou par un micro associé à un programme de reconnaissance vocale.

Selon un mode de réalisation préférentiel, le dispositif comprend au moins un ordinateur distant dudit équipement de visualisation, et des moyens de communication entre ledit équipement de visualisation et ledit ordinateur distant.

L'ordinateur distant peut, par exemple, réaliser le traitement et l'analyse des données collectées par l'équipement de visualisation. Les moyens de communication peuvent notamment être des moyens radio ou filaires.

Selon un mode de réalisation particulièrement avantageux, l'équipement de visualisation portable comprend au moins une surface semi-réfléchissante destinée à être placée devant au moins un œil de la personne, et au moins un dispositif d'illumination et/ou de captation d'images, pointé dans la direction dudit œil par l'intermédiaire d'une réflexion sur ladite surface semi-réfléchissante.

Ces dispositifs d'illumination et/ou de captage d'image peuvent ainsi être pointés sur l'œil de la personne, afin de l'observer ou d'y réaliser des examens, sans gêner la vision de l'écran par cet œil.

Avantageusement, ladite surface semi-réfléchissante est constituée par la surface dudit écran.

Selon un autre mode de réalisation avantageux, ladite surface semi-réfléchissante est placée devant la surface dudit écran.

Avantageusement, ledit dispositif d'illumination et/ou de captation d'images est une caméra.

Dans ce cas, le dispositif peux avantageusement réaliser un suivi de la position de l'œil.

Selon une autre solution avantageuse, ledit dispositif d'illumination et/ou de captation d'images comprend un émetteur laser et des moyens de captation de la réflexion sur l'œil du rayon laser émis par ledit émetteur laser.

Dans ce cas, le dispositif peut avantageusement réaliser des examens tels qu'une tomographie de cohérence optique.

### Liste des figures

D'autres objectifs et avantages de l'invention apparatront plus clairement à la lecture de la description suivante de modes de réalisation préférentiels, donnée à titre de simple exemple figuratif et non limitatif, et accompagnée de figures parmi lesquelles :
- la figure 1 est une représentation d'un équipement de visualisation adapté à la réalisation d'explorations du système visuel ;
- la figure 2 est une vue de détail d'un équipement de visualisation selon un mode de réalisation particulier;
- la figure 3 est une vue de coupe partielle schématique, selon un plan horizontal, de la tête d'une personne portant l'équipement de visualisation de la figure 2 ;
- la figure <3> est une représentation d'un équipement de visualisation selon <4> une variante de l'invention.

### Description détaillée de modes de réalisation de l'invention

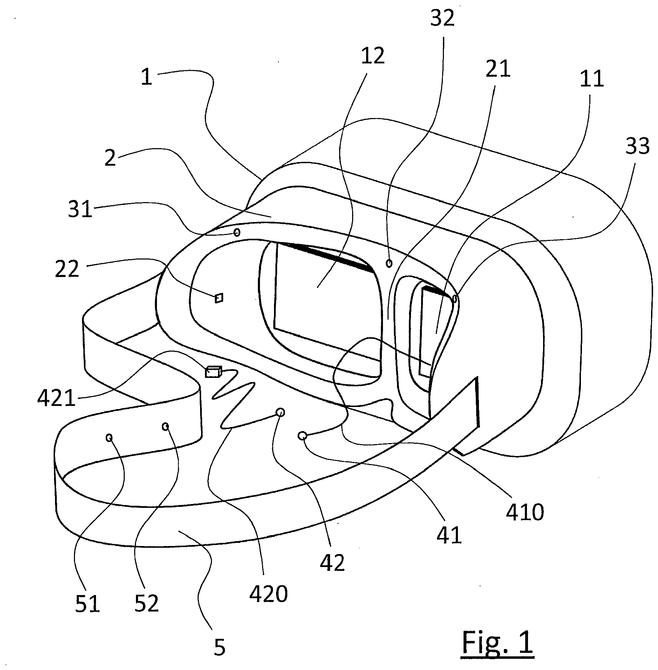

La figure 1 représente un équipement de visualisation portable adapté, selon un mode de réalisation de l'invention, pour la réalisation d'explorations du système visuel.

Dans la présente description, le terme « équipement de visualisation portable » désigne un équipement pouvant être porté sur le visage d'une personne et lui permettant la visualisation d'images sur un ou plusieurs écrans placés à une position fixe devant ses yeux, indépendamment des mouvements de la personne. De tels équipements de visualisation portable peuvent ainsi permettre la visualisation d'images différenciées par chacun des deux yeux de la personne, par exemple d'images présentant des angles de vue différents d'un même objet virtuel. De tels équipements sont connus en eux-mêmes et sont souvent intégrés à un casque, un masque ou à une paire de lunettes. Ils sont souvent utilisés pour permettre la visualisation de réalité virtuelle, ou d'images en trois dimensions.

Le dispositif représenté par la figure 1 comprend ainsi un masque 1 constituant un équipement de visualisation portable spécialement adapté pour la réalisation d'explorations du système visuel. Ce masque 1 présente un écran de visualisation droit 11, placé de façon à recouvrir l'œil droit de la personne et un écran de visualisation gauche 12 placé de façon à recouvrir l'œil gauche de la personne.

Une sangle 5 attachée au masque 1, à droite de l'écran de visualisation droit 11 d'une part et à gauche de l'écran de visualisation gauche 12 d'autre part, est destinée à permettre le maintien du masque 1 sur le visage de la personne. Cette sangle 5 peut avantageusement être une sangle élastique, et sa longueur est de préférence réglable, selon des procédés connus, pour permettre un bon ajustement et un bon maintien du masque 1 sur le visage.

De façon avantageuse, le masque 1 présente également des lèvres d'obturation 2, entourant les deux écrans de visualisation 11 et 12, et destinées à être plaquées contre la peau de la personne, autour de ses yeux. Ces lèvres d'obturation 2 permettent ainsi d'isoler les yeux de la personne de toute lumière extérieure, usuellement appelée lumière parasite ou lumière perturbatrice, ne provenant pas de l'un des écrans de visualisation 11 et 12. Le dispositif d'exploration du système visuel assure donc une maîrise parfaite de la lumière pouvant être vue par les yeux de la personne, indépendamment des conditions de lumière de la pièce dans laquelle la personne est placée, ce qui permet une exploration du système visuel dans des conditions parfaitement ma Irisées.

De façon avantageuse, les lèvres d'obturation 2 présentent une branche centrale 21, destinée à être plaquée contre le front et le nez de la personne, et permettant d'isoler chacun des yeux de la personne de la lumière émise par l'écran de visualisation placé en face de l'autre œil.Cette branche centrale 21 permet une maîtrise parfaite de la lumière arrivant à chaque œil de la personne, ce qui donne la possibilité de réaliser une exploration du système visuel mettant en œuvre des stimulations de chaque œil, indépendamment.

Grâce à la présence de ces lèvres d'obturation, le masque 1 peut placer les yeux de la personne dans l'obscurité totale, indépendamment de la luminosité de la pièce dans laquelle il est situé. Le masque 1, porté par la personne, permet donc son adaptation à l'obscurité pendant vingt à trente minutes, puis la réalisation de l'électrorétinogramme, sans qu'il ne soit pour autant situé dans une pièce obscure.

Selon un premier mode de réalisation possible, les lèvres d'obturation 2 peuvent être constituées par une paroi de matériau souple, tel qu'un caoutchouc souple, un silicone ou toute autre matière souple appropriée. Préférentiellement, cette paroi est de couleur sombre et d'épaisseur suffisante pour empêcher le passage de la lumière. Le bord libre de cette paroi vient, en utilisation, se plaquer contre la peau de la personne, en se déformant pour épouser la forme de son visage.

Selon un autre mode de réalisation possible, les lèvres d'obturation sont constituées de mousse souple, préférentiellement de couleur sombre et suffisamment épaisse pour empêcher le passage de la lumière. Cette mousse peut être recouverte par un film plastique souple, ou un tissu. Le bord libre de ces lèvres vient, en utilisation, se plaquer contre la peau de la personne, en se déformant pour épouser la forme de son visage.

Il est à noter que, dans d'autres modes de réalisation possibles de l'invention, le masque 1 peut présenter un unique écran, placé de façon à recouvrir les deux yeux de la personne. Dans ce cas, les lèvres d'obturation 2 peuvent entourer l'écran unique, sans présenter de branche centrale. Il est aussi possible qu'une branche centrale s'étende devant cet écran, afin de le séparer en deux portions placées chacune devant l'un des yeux de la personne.

De préférence, les portions des lèvres d'obturation 2 destinées à être en contact avec la peau de la personne peuvent comprendre une ou plusieurs électrodes de référence 31, 32 et 33. Ces électrodes peuvent avantageusement être des électrodes de contact sèches. Ainsi, lorsque la personne porte le masque 1, les électrodes de référence 31, 32 et 33 sont placées directement contre sa peau, sans qu'il soit nécessaire de les installer séparément du masque 1 ou de les coller. Dans le mode de réalisation représenté, les lèvres 2 présentent trois électrodes de référence 31, 32 et 33. Le nombre et la position de ces électrodes de référence peuvent cependant être différents, dans d'autres modes de réalisation de l'invention.

### Électrodes de mesure.

Les électrodes de mesure 41 et 42, ou électrodes oculaires, permettant de réaliser un électrorétinogramme, doivent être placées en contact ou à proximité des yeux de la personne. Préférentiellement, pour obtenir un signal présentant un bon rapport signal sur bruit, ces électrodes doivent être placées au contact de l'oeil, souvent sous une des paupières de la personne. Quand ce placement sur l'œil est impossible ou compliqué, il est également possible de les placer sur une paupière, par exemple sur la paupière inférieure de la personne. Ces électrodes doivent être placées par un opérateur, avant que le masque 1 ne soit positionné sur les yeux de la personne.

De façon avantageuse, ces électrodes de mesure 41 et 42 sont reliées au masque 1 par des fils, respectivement 410 et 420. Comme le montre la figure 1, l'extrémité du fil 420 qui n'est pas reliée à l'électrode 42 est connectée à une prise mâle 421, dont la forme est complémentaire d'une prise femelle 22 formant un connecteur. Cette prise femelle 22 est placée, dans le masque 1, dans l'espace situé à l'intérieur des lèvres d'obturation 2, c'est-à-dire entre l'un des écrans de visualisation et le bord libre des lèvres d'obturation 2 entourant cet écran. De préférence, comme le représente la figure 1, cette prise femelle 22 est située dans la face interne de l'une des lèvres 2. Dans d'autres modes de réalisation, cette prise peut être située à côté de l'écran 11 ou 12, dans la zone entourée par les lèvres 2.

Ainsi, quand une personne porte le masque 2, ce fil 420 ne passe pas entre la peau de la personne et la lèvre d'obturation 2. Il n'y a du coup aucun soulèvement local de la lèvre 2, qui pourrait permettre la pénétration de lumière extérieure vers l'œil de la personne. Ce branchement particulier de l'électrode 42 contribue donc à la maîtrise parfaite de la lumière arrivant à l'œil, et des conditions de l'exploration de l'œil de la personne.

Le fil 410 de l'électrode de mesure 41 est avantageusement branché de façon identique, dans un connecteur adapté placé dans l'espace situé à l'intérieur des lèvres d'obturation 2.

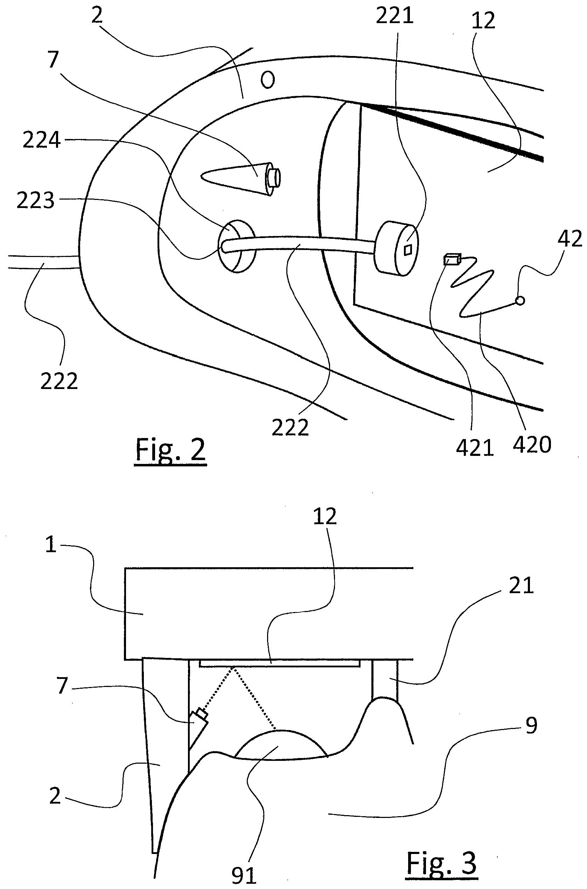

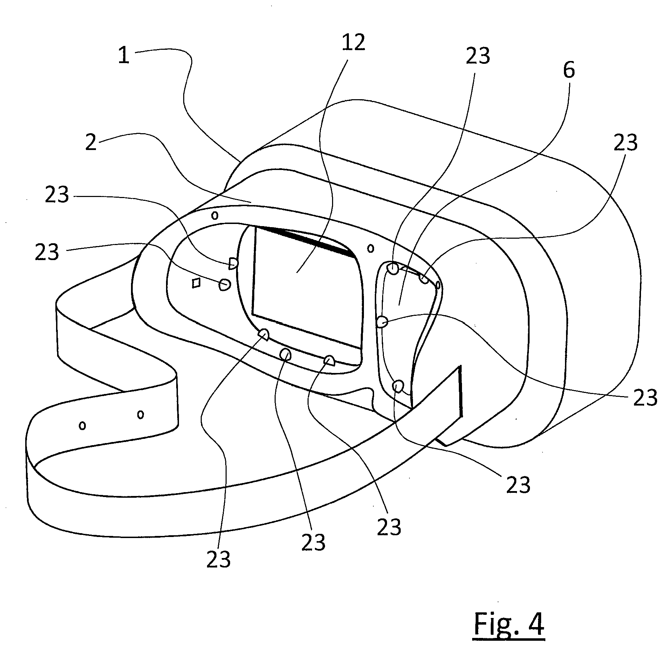

La figure 2 est une vue de détail d'un masque 1, constituant un équipement de visualisation portable, qui est adapté selon un mode de réalisation particulier. Cette figure montre ainsi une portion des lèvres d'obturation 2 de ce masque, dans laquelle un trou cylindrique 223 a été percé pour laisser le passage à un fil 222. L'extrémité de ce fil située à l'intérieur de l'espace défini par les lèvres d'obturation porte une prise femelle 221, formant un connecteur dans lequel peut se connecter la prise mâle 421 reliée à l'électrode 42 par le fil 420.

La longueur du fil 222 qui est situé à l'intérieur de l'espace défini par les lèvres d'obturation 2 permet de réaliser plus facilement le branchement de la prise mâle 421 dans la prise femelle 221, après que l'opérateur ait installé l'électrode 42 sur l'œil de la personne, et avant de positionner le masque 1 sur le visage de la personne. Une fois ce branchement effectué, et le masque 1 positionné sur le visage de la personne, il est possible de tirer sur le fil 222, depuis l'extérieur du masque 1, jusqu'à ce que la prise femelle 221 vienne se placer dans un espace 224 prévu pour la recevoir, sur la face interne de la lèvre d'obturation 2, autour du trou 223. Ainsi, le fil 222 ne reste pas dans l'espace situé à l'intérieur des lèvres d'obturation 2, et ne gêne pas la vision, par la personne, de l'écran 12 du masque 1. Par ailleurs, le trou 223 est avantageusement d'un diamètre sensiblement égal au diamètre du fil 222, de telle sorte que la lumière externe ne peut pas traverser la lèvre 2 par ce trou 223.

La figure <3> représente un masque 1 constituant un équipement de visualisation <4> portable selon une variante de l'invention. Selon cette variante, la face interne de la lèvre 2 présente des ergots 23 permettant le maintien de lentilles optiques 6, ou verres correcteurs, devant les écrans 11 et 12. Dans la variante réalisée, seule une lentille 6 a été placée devant l'écran 11. De préférence, la personne utilisant le masque 1, ou un opérateur, peut placer dans les ergots 23 des lentilles 6 présentant une correction adaptée aux yeux de la personne, pour qu'elle voie de façon nette les images projetées par les écrans 11 et 12.

De façon particulièrement avantageuse, le masque 1 peut comprendre des moyens permettant de mesurer la correction adaptée. Ainsi, le masque 1 peut comprendre des moyens permettant d'effectuer une analyse par réfractométrie de l'œil de la personne, afin de déterminer la correction nécessaire à apporter.

Selon encore une autre variante, les écrans 11 et 12 peuvent être adaptés pour afficher des images simulant la correction optique nécessaire, pour que la personne les voie de façon nette. Une telle adaptation des écrans 11 et 12 est connue, en elle-même, de l'homme du métier.

L'utilisation d'un équipement de visualisation portable adapté selon l'invention permet avantageusement de réaliser un électrorétinogramme de façon plus simple qu'avec les solutions de l'art antérieur.

Pour réaliser ce test, un opérateur doit préalablement poser les électrodes de mesure, soit en contact avec l'œil de la personne, soit en contact avec sa paupière inférieure. Après avoir installé ces électrodes de mesure, il peut brancher les fils de ces électrodes aux connecteurs situés dans le masque 1, et mettre en place le masque sur les yeux de la personne, en s'assurant que les fils des électrodes de mesure sont bien placés à l'intérieur des lèvres d'obturation et ne gênent pas la vision, par la personne, des écrans 11 et 12.

Lors de cette installation du masque 1, les électrodes de référence 31, 32 et 33 portées par les lèvres d'obturation 2 sont directement positionnées en contact avec la peau de la personne, autour de ses yeux.

Une fois le masque 1 installé, les yeux de la personne sont maintenus dans l'obscurité par le masque, les écrans 11 et 12 et les lèvres d'obturation 2 les isolant de la lumière extérieure. La personne peut ainsi être maintenue dans l'obscurité pendant une période de vingt à trente minutes afin de réaliser son adaptation à l'obscurité, se traduisant par une dilatation stable des pupilles.

Après cette étape d'adaptation à l'obscurité, l'électrorétinogramme lui-même est effectué sans que la personne n'ait besoin de retirer le masque, ni de changer de position. Pour cela, les écrans de visualisation 11 et 12 affichent des stimulations visuelles ou des flashs lumineux. Ces stimulations peuvent être monoculaires, c'est-à-dire devant un seul des yeux de la personne, par l'intermédiaire d'un seul des écrans 11 ou 12, ou binoculaires par une stimulation simultanée des deux yeux, effectuée simultanément par les écrans 11 et 12. Dans les modes de réalisation dans lesquels le masque 1 ne présente qu'un unique écran s'étendant devant les deux yeux de la personne, une stimulation monoculaire peut par exemple être obtenue en plaçant un cache opaque devant l'un des yeux de la personne. Ce cache opaque peut par exemple être maintenu par des ergots 23 prévus pour le maintien de lentilles optiques.

Ces stimulations visuelles peuvent être celles qui sont utilisées dans les protocoles d'examen connus. Elles peuvent ainsi consister en des flash lumineux, ou en l'affichage de motifs déterminés, stables ou variables, à une distance déterminée des yeux de la personne.

Dans le cas d'une stimulation monoculaire, la distance entre l'image et l'œil de la personne peut être simulée par une adaptation de la taille de l'image affichée sur l'écran. Dans le cas d'une stimulation binoculaire, cette distance peut être simulée par une adaptation de la taille de l'image et par un décalage d'angle de vue, entre les images affichées sur les écrans 11 et 12, permettant une vision stéréoscopique.

De façon avantageuse, dans le cadre d'un protocole de stimulation correspondant à un électrorétinogramme multifocal, les images affichées peuvent changer à un rythme élevé, afin de stimuler séquentiellement des zones différentes de la rétine. De la même façon, les protocoles peuvent mettre en œuvre des stimulations visuelles constituées d'images en noir et blanc ou en couleurs, afin de stimuler différents neurones rétiniens. De même, l'usage d'un équipement de visualisation portable permet également des protocoles mettant en oeuvre des stimulations lumineuses sous forme d'images en trois dimensions.

Un système d'acquisition, avantageusement porté par le masque 1, permet de synchroniser les données mesurées par les électrodes avec les stimulations visuelles produites par les écrans 11 et 12, afin d'obtenir des mesures d'électrorétinogramme utiles. Ce système d'acquisition comporte de préférence des moyens d'amplification des signaux mesurés par les électrodes, des moyens de filtrage de ces signaux pour en supprimer les composantes non pertinentes, et des moyens de conversion de ces signaux analogiques en des signaux numériques.

Les moyens d'amplification peuvent par exemple comprendre un préamplificateur d'instrumentation et d'isolement dont le gain est d'au moins 80dB à haut taux de réjection de mode commun (supérieur à 100dB), et un amplificateur de mise à l'échelle dont le gain est ajusté avant conversion. Les moyens de filtrage peuvent comprendre un filtre passe-haut dont la fréquence de coupure est d'environ 0,2 Hz, pour rejeter les composantes continues et très lentement variables, un filtre passe-bas pour éliminer les perturbations hautes fréquences au-delà de 300hz et des filtres numériques, mis en œuvre après la conversion analogique-numérique, pour la réjection des potentiels à 50 Hz dus au courant électrique alternatif du secteur. Les moyens de conversion peuvent comprendre un convertisseur analogique-numérique de huit voies analogiques, à 12 bits de conversion et dont la fréquence d'échantillonnage peut être fixée à 4 096 échantillons par seconde.

Les données ayant été ainsi traitées par le système d'acquisition peuvent alors être envoyées à un logiciel de traitement, apte à réaliser des interprétations de ces données. Selon un mode de réalisation préférentiel, ce logiciel de traitement est mis en œuvre sur un ordinateur distant du masque 1. Dans ce cas, le masque 1 peut comprendre des moyens de communication, par exemple par radio, lui permettant d'envoyer les données mesurées et traitées par le système d'acquisition vers l'ordinateur sur lequel est mis en œuvre le logiciel de traitement.

Avantageusement, un logiciel peut être mis en œuvre dans le masque 1, ou dans un ordinateur connecté au masque 1, pour gérer la succession d'étapes nécessaires pour réaliser l'exploration du système visuel. Ainsi, ce logiciel peut gérer une sortie audio constituée par un haut-parleur, des écouteurs, ou par une prise permettant le branchement d'un haut-parleur ou d'écouteurs, apte à communiquer à la personne utilisant le masque 1 des instructions d'utilisation pour la réalisation correcte de l'exploration. Ce logiciel peut également commander l'affichage d'informations, soit sur l'un des écrans 11 et 12 pour qu'elles soient visibles de la personne portant le masque 1, soit sur un autre écran porté par le masque 1, placé de façon à être visible d'une personne ne portant pas le masque, comme un opérateur.

Selon une solution avantageuse, la personne portant le masque 1 peut interagir avec le logiciel, par le biais d'un périphérique d'entrée adapté. Ce périphérique d'entrée peut par exemple être constitué par une manette de commande (usuellement désignée par le terme anglais « joystick »), qui peut être connectée au masque 1 par un câble de connexion ou par une liaison radio, par exemple selon le protocole « Bluetooth ». Ce périphérique d'entrée peut également être constitué par un micro, par exemple placé dans le masque 1, pouvant capter la voix de la personne. Dans ce cas, le logiciel peut comprendre des moyens de reconnaissance vocale, permettant par exemple de reconnaître des instructions simples de l'utilisateur.

Ce logiciel peut également effectuer une analyse préliminaire des données recueillies et traitées par le système d'acquisition, afin de vérifier la cohérence des données mesurées par les électrodes. Il peut ainsi vérifier, avant toute stimulation visuelle, que les électrodes mesurent un signal montrant qu'elles sont correctement positionnées. Il peut ensuite mettre en œuvre les séries de stimulations visuelles, et analyser les données recueillies et traitées par le système d'acquisition afin de vérifier que ces données ne sont pas perturbées par des anomalies de mesure, telles qu'un mouvement des yeux ou des paupières de la personne. Si de telles anomalies apparaissent, le logiciel peut répéter une série de stimulations, afin de tenter d'obtenir, pour ces stimulations, un résultat exempt d'anomalies.

Ainsi, le logiciel peut coordonner la réalisation successive ou simultanée d'une série d'étapes ou d'examens, sans qu'une intervention d'un opérateur ne soit nécessaire. Dans le cas d'un électrorétinogramme, par exemple, le logiciel peut démarrer automatiquement les étapes de stimulation, après l'étape d'adaptation à l'obscurité.

L'équipement de visualisation portable selon l'invention peut, avantageusement, comprendre des composants lui permettant de réaliser d'autres examens visant à l'exploration du système visuel ou neurologique.

Par exemple, dans un mode de réalisation particulièrement avantageux, la sangle de maintien 5 peut présenter une ou plusieurs électrodes, par exemple deux électrodes occipitales droite 51 et gauche 52, destinées à mesurer les potentiels électrophysiologiques dans les populations de neurones occipitaux situées à l'arrière du crâne, juste au-dessus de la nuque. Ces électrodes peuvent avantageusement être des électrodes de contact sèches. Les données mesurées par ces électrodes occipitales 51 et 52 peuvent avantageusement être recueillies et traitées par le système d'acquisition recueillant les données provenant des électrodes de référence et des électrodes oculaires. De préférence, le système d'acquisition recueille simultanément les données provenant des électrodes rétiniennes 41 et 42 et des électrodes occipitales 51 et 52, afin de mesurer de façon synchrone la réponse du cortex occipital aux stimulations lumineuses perçues par la rétine.

Dans un autre mode de réalisation possible, l'équipement de visualisation peut comprendre des moyens de mesure du champ visuel de la personne, par exemple avant la réalisation de l'électrorétinogramme. Pour cela, les écrans 11 et 12 peuvent afficher des points lumineux à des positions successives déterminées, mais non prévisibles par la personne, dans et en dehors de son champ visuel, la personne indiquant si elle perçoit ces points, par exemple par l'intermédiaire d'une manette de commande ou par des instructions orales, perçues par un micro et interprétées par un logiciel de l'équipement de visualisation.

L'équipement de visualisation peut également comprendre, de façon utile, des moyens d'exploration permettant de réaliser une Tomographie en Cohérence Optique (usuellement désignée par l'acronyme « OCT »), une rétinophotographie, une rétinorefractométrie ou une pupillométrie. Une pupillométrie peut ainsi être réalisée avant un électrorétinogramme, pour vérifier que la pupille est suffisamment dilatée pour réaliser l'électrorétinogramme, et pendant ou après l'électrorétinogramme, pour vérifier que la dilatation reste constante. Il est également possible de détecter la position de l'œil et/ou des paupières (opération couramment désignée par l'expression anglaise « eye tracking »), par exemple pour assurer un contrôle total des conditions optimales d'examen.

Pour réaliser de tels examens ou une détection de la position de l'œil, il est connu d'utiliser un système d'illumination, par exemple un rayon laser, et/ou un système de captation d'image, tel qu'une caméra ou un appareil photo, qui doivent être pointés sur l'œil de la personne. Dans un équipement de visualisation portable, la présence d'un ou de plusieurs écrans en face des yeux de la personne empêche cependant de placer un tel système d'illumination ou système de captation d'images dans une position appropriée, en face des yeux de la personne.

Selon une caractéristique particulièrement avantageuse de l'invention, il a été imaginé de placer de tels systèmes d'illumination ou de captation d'images en dehors du champ de vision de la personne, et de les pointer sur un œil par l'intermédiaire d'une réflexion sur une surface semi-réfléchissante, placée en face d'un œil ou des yeux de la personne. Cette surface semi-réfléchissante peut par exemple être constituée par la surface de l'écran lui-même, ou toute autre surface semi-réfléchissante placée devant l'écran. Elle peut, si nécessaire, présenter une orientation particulière distincte de l'orientation de l'écran. Cette surface semi-réfléchissante ne gêne pas la vision de l'écran, mais elle permet, par exemple quand l'écran n'émet pas ou peu de lumière, de réfléchir un rayon lumineux tel qu'un rayon laser pour le pointer sur l'œil, ou de réfléchir une image de l'œil pour la capter sur une caméra ou un appareil photo.

Les figures 2 et 3 illustrent un mode de réalisation de cette solution technique. Sur la figure 2, on peut voir que les lèvres d'obturation 2 du maque 1 portent une caméra 7 miniaturisée, qui est pointée vers l'écran 12. Cette caméra 7 est également visible sur la figure 3, qui est une vue de coupe schématique, selon un plan horizontal, montrant une partie de la tête 9 d'une personne portant le masque 1. Comme le montrent les pointillés représentant le pointage de la caméra 7, celle-ci permet de capter des images de l'œil 91, qui se reflètent dans l'écran 12 constituant une surface semi-réfléchissante. La caméra 7 peut ainsi, par exemple, capter des images représentatives de la position de l'œil, de la taille de la pupille, ou de la position des paupières.

L'utilisation de cette caméra peut par exemple être faite à un moment où l'écran 7 est éteint, ou à un moment ou cet écran n'émet qu'une lumière faible et uniforme, suffisante pour éclairer l'œil mais ne perturbant pas la réflexion de l'image de l'œil, ou encore à un moment où seule une zone de l'écran non utilisée pour la réflexion émet de la lumière, afin d'éclairer l'œil, la zone de l'écran utilisée pour la réflexion étant éteinte. Dans d'autres cas, l'éclairage permettant l'utilisation de la caméra peut être fait par l'intermédiaire de diodes électroluminescentes placées à côté des écrans.

Dans d'autres modes de réalisation, il est possible de pointer un rayon laser sur l'œil, par l'intermédiaire d'une réflexion sur l'écran ou sur une surface semi-réfléchissante placée devant l'écran, et de capter de la même manière la réflexion par l'œil de ce rayon laser.

Il est à noter qu'une telle utilisation de l'écran placé devant un œil comme surface semi-réfléchissante, ou l'utilisation d'une surface semi-réfléchissante placée devant l'écran, afin de pointer sur l'œil un système d'illumination ou un système de captation d'image, peut être avantageusement mis en œuvre dans tout type d'équipement de visualisation portable comprenant au moins un écran de visualisation, destiné à être placé face à au moins un œil d'une personne, indépendamment des autres caractéristiques de l'équipement de visualisation décrites dans la présente demande.

Un équipement portable de visualisation, comprenant au moins un écran de visualisation destiné à être placé face à au moins un œil d'une personne, et comprenant au moins une surface semi-réfléchissante destinée à être placée devant au moins un œil de la personne, et au moins un dispositif d'illumination et/ou de captation d'image, pointé sur l'œil par l'intermédiaire d'une réflexion sur la surface semi-réfléchissante, est ainsi particulièrement avantageux.

Dans ce cas, la surface semi-réfléchissante peut avantageusement être soit la surface de l'écran lui-même, soit une surface semi-réfléchissante placée devant l'écran.

## Revendications

1. Dispositif d'exploration du système visuel, comprenant un équipement de visualisation portable (1) comportant au moins un écran de visualisation (11, 12) destiné à être placé face à au moins un œil d'une personne,
edit équipement de visualisation (1) comprenant des lèvres d'obturation (2) entourant au moins ledit ou l'un desdits écrans de visualisation (11, 12), et présentant un bord libre destiné à être appliqué contre la peau de la personne, autour de son œil ou de ses yeux faisant face audit écran (11, 12), ledit bord libre comprenant au moins une électrode (31, 32, 33) de mesure du potentiel électrique, destinée à être appliquée contre la peau de la personne,
**caractérisé en ce que** ledit dispositif comprend au moins une électrode libre (41, 42) de mesure du potentiel électrique, reliée, par un fil de connexion (410, 420), à un connecteur (22) porté par ledit équipement de visualisation (1), entre l'un desdits écrans de visualisation (11, 12) et ledit bord libre des lèvres d'obturation (2) entourant cet écran (11, 12).

2. Dispositif d'exploration selon la revendication précédente, **caractérisé en ce que** ledit équipement de visualisation (1) comprend deux écrans de visualisation (11, 12), destinés chacun à être placé face à l'un des yeux de ladite personne, et **en ce que** lesdites lèvres d'obturation (2, 21) entourent chacun desdits écrans (11, 12).

3. Dispositif d'exploration selon l'une quelconque des revendications précédentes, **caractérisé en ce que** lesdites lèvres d'obturation (2, 21) comprennent des moyens de maintien (23) de lentilles optiques (6) devant au moins un desdits écrans (11, 12).

4. Dispositif d'exploration selon l'une quelconque des revendications précédentes, **caractérisé en ce qu'**il comprend une sangle (5) de maintien dudit équipement de visualisation (1), destinée à être passée à l'arrière de la tête de ladite personne, ladite sangle de maintien portant au moins une électrode (51, 52)de mesure du potentiel électrique.

5. Dispositif d'exploration selon l'une quelconque des revendications précédentes, **caractérisé en ce que** ledit connecteur (22) est porté par ledit équipement de visualisation (1) par l'intermédiaire d'un fil (222) de longueur variable.

6. Dispositif d'exploration selon l'une quelconque des revendications précédentes, **caractérisé en ce qu'**il comprend un système d'acquisition apte à synchroniser les données mesurées par les électrodes (31, 32, 33, 41, 42, 51, 52) avec les stimulations visuelles produites par ledit ou lesdits écrans (11, 12).

7. Dispositif d'exploration selon la revendication précédente, **caractérisé en ce que** ledit système d'acquisition comporte des moyens d'amplification des signaux mesurés par les électrodes (31, 32, 33, 41, 42, 51, 52), des moyens de filtrage de ces signaux, et des moyens de conversion de ces signaux analogiques en des signaux numériques.

8. Dispositif d'exploration selon l'une quelconque des revendications précédentes, **caractérisé en ce qu'**il comprend une sortie audio apte à communiquer des instructions à ladite personne.

9. Dispositif d'exploration selon l'une quelconque des revendications précédentes, **caractérisé en ce qu'**il comprend au moins un périphérique d'entrée.

10. Dispositif d'exploration selon l'une quelconque des revendications précédentes, **caractérisé en ce qu'**il comprend au moins un ordinateur distant dudit équipement de visualisation (1), et des moyens de communication entre ledit équipement de visualisation (1) et ledit ordinateur distant.

11. Dispositif d'exploration selon l'une quelconque des revendications précédentes, **caractérisé en ce que** ledit équipement de visualisation portable (1) comprend au moins une surface semi-réfléchissante destinée à être placée devant au moins un œil (91) de la personne, et au moins un dispositif d'illumination et/ou de captation d'images (7), pointé dans la direction dudit œil par l'intermédiaire d'une réflexion sur ladite surface semi-réfléchissante.

12. Dispositif d'exploration selon la revendication précédente, **caractérisé en ce que** ladite surface semi-réfléchissante est constituée par la surface dudit écran (12).

13. Dispositif d'exploration selon la revendication 11, **caractérisé en ce que** ladite surface semi-réfléchissante est placée devant la surface dudit écran (12).

14. Dispositif d'exploration selon l'une quelconque des revendications 11 à 13, **caractérisé en ce que** ledit dispositif d'illumination et/ou de captation d'images est une caméra (7).

15. Dispositif d'exploration selon l'une quelconque des revendications 11 à 13, **caractérisé en ce que** ledit dispositif d'illumination et/ou de captation d'images comprend un émetteur laser et des moyens de captation de la réflexion sur l'œil du rayon laser émis par ledit émetteur laser.

## Patentansprüche

1. Vorrichtung zum Untersuchen des visuellen Systems, die eine tragbare Anzeigeeinrichtung (1) umfasst, die wenigstens einen Anzeigebildschirm (11, 12) aufweist, der dafür bestimmt ist, gegenüber wenigstens einem Auge einer Person angeordnet zu werden, wobei die Anzeigeeinrichtung (1) Dichtlippen (2) umfasst, die wenigstens den oder einen der Anzeigebildschirme (11, 12) umgeben und einen freien Rand aufweisen, der dafür bestimmt ist, an die Haut der Person um ihr Auge oder ihre Augen herum, die dem Bildschirm (11, 12) gegenüberliegen, angelegt zu werden, wobei der freie Rand wenigstens eine Elektrode (31, 32, 33) zum Messen des elektrischen Potentials umfasst, die dafür bestimmt ist, an die Haut der Person angelegt zu werden, **dadurch gekennzeichnet, dass** die Vorrichtung wenigstens eine freie Elektrode (41, 42) zum Messen des elektrischen Potentials umfasst, die zwischen einem der Anzeigebildschirme (11, 12) und dem freien Rand der Dichtlippen (2), die diesen Bildschirm (11, 12) umgeben, durch einen Verbindungsdraht (410, 420) mit einem an der Anzeigeeinrichtung (1) befestigten Verbinder (22) verbunden ist.

2. Untersuchungsvorrichtung nach dem vorhergehenden Anspruch, **dadurch gekennzeichnet, dass** die Anzeigeeinrichtung (1) zwei Anzeigebildschirme (11, 12) umfasst, die jeweils dafür bestimmt sind, gegenüber einem der Augen der Person angeordnet zu werden, und dass die Dichtlippen (2, 21) jeden der Bildschirme (11, 12) umgeben.

3. Untersuchungsvorrichtung nach einem der vorhergehenden Ansprüche, **dadurch gekennzeichnet, dass** die Dichtlippen (2, 21) Mittel (23) zum Halten von optischen Linsen (6) vor wenigstens einem der Bildschirme (11, 12) umfassen.

4. Untersuchungsvorrichtung nach einem der vorhergehenden Ansprüche, **dadurch gekennzeichnet, dass** sie einen Riemen (5) zum Halten der Anzeigeeinrichtung (1) umfasst, der dafür bestimmt ist, über den Hinterkopf der Person geführt zu werden, wobei an dem Halteriemen wenigstens eine Elektrode (51, 52) zum Messen des elektrischen Potentials befestigt ist.

5. Untersuchungsvorrichtung nach einem der vorhergehenden Ansprüche, **dadurch gekennzeichnet, dass** der Verbinder (22) an der Anzeigeeinrichtung (1) über einen Draht (222) mit variabler Länge befestigt ist.

6. Untersuchungsvorrichtung nach einem der vorhergehenden Ansprüche, **dadurch gekennzeichnet, dass** sie ein Erfassungssystem umfasst, das geeignet ist, die durch die Elektroden (31, 32, 33, 41, 42, 51, 52) gemessenen Daten mit den durch den oder die Bildschirme (11, 12) erzeugten visuellen Stimulationen zu synchronisieren.

7. Untersuchungsvorrichtung nach dem vorhergehenden Anspruch, **dadurch gekennzeichnet, dass** das Erfassungssystem Mittel zum Verstärken der durch die Elektroden (31, 32, 33, 41, 42, 51, 52) gemessenen Signale, Mittel zum Filtern dieser Signale und Mittel zum Umwandeln dieser analogen Signale in digitale Signale aufweist.

8. Untersuchungsvorrichtung nach einem der vorhergehenden Ansprüche, **dadurch gekennzeichnet, dass** sie einen Audioausgang umfasst, der geeignet ist, Anweisungen an die Person zu übermitteln.

9. Untersuchungsvorrichtung nach einem der vorhergehenden Ansprüche, **dadurch gekennzeichnet, dass** sie wenigstens ein Eingabegerät umfasst.

10. Untersuchungsvorrichtung nach einem der vorhergehenden Ansprüche, **dadurch gekennzeichnet, dass** sie wenigstens einen Computer, der von der Anzeigeeinrichtung (1) entfernt ist, und Kommunikationsmittel zwischen der Anzeigeeinrichtung (1) und dem entfernten Computer umfasst.

11. Untersuchungsvorrichtung nach einem der vorhergehenden Ansprüche, **dadurch gekennzeichnet, dass** die tragbare Anzeigeeinrichtung (1) wenigstens eine halbreflektierende Oberfläche, die dafür bestimmt ist, vor wenigstens einem Auge (91) der Person angeordnet zu werden, und wenigstens eine Beleuchtungs- und/oder Bildaufnahmevorrichtung (7) umfasst, die über eine Reflexion an der halbreflektierenden Oberfläche in die Richtung des Auges gerichtet ist.

12. Untersuchungsvorrichtung nach dem vorhergehenden Anspruch, **dadurch gekennzeichnet, dass** die halbreflektierende Oberfläche durch die Oberfläche des Bildschirms (12) gebildet wird.

13. Untersuchungsvorrichtung nach Anspruch 11, **dadurch gekennzeichnet, dass** die halbreflektierende Oberfläche vor der Oberfläche des Bildschirms (12) angeordnet ist.

14. Untersuchungsvorrichtung nach einem der Ansprüche 11 bis 13, **dadurch gekennzeichnet, dass** die Beleuchtungs- und/oder Bildaufnahmevorrichtung eine Kamera (7) ist.

15. Untersuchungsvorrichtung nach einem der Ansprüche 11 bis 13, **dadurch gekennzeichnet, dass** die Beleuchtungs- und/oder Bildaufnahmevorrichtung einen Laseremitter und Mittel zum Aufnehmen der Reflexion des durch den Laseremitter emittierten Laserstrahls auf dem Auge umfasst.

## Claims

1. Device for exploration of the visual system, comprising portable display equipment (1) comprising at least one display screen (11, 12) intended to be placed in front of at least one eye of a person,
said display equipment (1) comprises closure lips (2) that surround at least said display screen or one of said display screens (11, 12) and comprise a free edge that is intended to be applied against the person's skin, around his eye or his eyes in front of said screen (11, 12), said free edge comprising at least one electrode (31, 32, 33) for measuring the electrical potential, which electrode is intended to be applied against the person's skin,
**characterized in that** said device comprise at least one free electrode (41, 42) for measuring the electrical potential, which electrode is connected, by means of a connecting wire (410, 420), to a connector (22) that is borne by said display equipment (1), between one of said display screens (11, 12) and said free edge of the closure lips (2) surrounding said screen (11, 12).

2. Exploration device according to the preceding claim, **characterized in that** said display equipment (1) comprises two display screens (11, 12) which are each intended to be placed in front of one of the eyes of said person, and **in that** said closure lips (2, 21) surround each of said screens (11, 12).

3. Exploration device according to either of the preceding claims, **characterized in that** said closure lips (2, 21) comprise means (23) for retaining optical lenses (6) in front of at least one of said screens (11, 12).

4. Exploration device according to any of the preceding claims, **characterized in that** it comprises a retaining strap (5) for said display equipment (1), which strap is intended to be passed behind the head of said person, said retaining strap bearing at least one electrode (51, 52) for measuring the electrical potential.

5. Exploration device according to any of the preceding claims, **characterized in that** said connector (22) is borne by said display equipment (1), by means of a wire (222) of variable length.

6. Exploration device according to any of the preceding claims, **characterized in that** it comprises an acquisition system that is capable of synchronizing the data measured by the electrodes (31, 32, 33, 41, 42, 51, 52) with the visual stimulations produced by said screen(s) (11, 12).

7. Exploration device according to the preceding claim, **characterized in that** said acquisition system comprises means for amplifying the signals measured by the electrodes (31, 32, 33, 41, 42, 51, 52), means for filtering said signals, and means for converting said analog signals into digital signals.

8. Exploration device according to any of the preceding claims, **characterized in that** it comprises an audio output that is capable of communicating instructions to said person.

9. Exploration device according to any of the preceding claims, **characterized in that** it comprises at least one input peripheral.

10. Exploration device according to any of the preceding claims, **characterized in that** it comprises at least one computer that is remote from said display equipment (1), and means for communicating between said display equipment (1) and said remote computer.

11. Exploration device according to any of the preceding claims, **characterized in that** said portable display equipment (1) comprises at least one semi-reflective surface that is intended to be placed in front of at least one eye (91) of the person, and at least one illumination and/or image capture device (7), pointed in the direction of said eye, by means of a reflection on said semi-reflective surface.

12. Exploration device according to the preceding claim, **characterized in that** said semi-reflective surface is formed by the surface of said screen (12).

13. Exploration device according to claim 11, **characterized in that** said semi-reflective surface is placed in front of the surface of said screen (12).

14. Exploration device according to any of claims 11 to 13, **characterized in that** said illumination and/or image capture device is a camera (7).

15. Exploration device according to any of claims 11 to 13, **characterized in that** said illumination and/or image capture device comprises a laser emitter and means for capturing the reflection, on the eye, of the laser beam emitted by said laser emitter.
